# EUROPÄISCHE PATENTANMELDUNG

(11) **EP 4 261 862 A1**
(43) Veröffentlichungstag der Anmeldung: **18.10.2023**
(21) Anmeldenummer: 23166967.2
(22) Anmeldetag: 06.04.2023
(51) Int. Cl.: H01H 3/14, A61B 8/00

(54) **MEDIZINTECHNISCHES GERÄT MIT DRAHTLOS ANGEBUNDENEM FUSSSCHALTER**

(30) Priorität: 11.04.2022 US 202263329735 P
(71) Anmelder: Olympus Winter & Ibe GmbH, 22045 Hamburg (DE)
(72) Erfinder: Wolter, Michael, 22307 Hamburg (DE)
(74) Vertreter: Glawe, Delfs, Moll

(57) **Zusammenfassung**

Die Erfindung betrifft ein System (1) umfassend ein über einen Fußschalter (20) steuerbares medizintechnisches Gerät (10) und einen Fußschalter (20), wobei der Fußschalter (20) einen Transmitter und das medizintechnische Gerät (10) einen Receiver zur drahtlosen Übermittlung von eindeutig zuordenbaren Signalen zur Steuerung vom Fußschalter (20) zum medizintechnischen Gerät (10) umfasst, und wobei der Receiver des medizintechnischen Geräts (10) zur Messung der Signalstärke von Signalen des Fußschalters (20) und das medizintechnische Gerät (10) zur Ausgabe einer Warnung, sofern die gemessene Signalstärke oder das Signal-Rausch-Verhältnis unter einem vorgegebenen Grenzwert liegt, ausgebildet ist.

## Beschreibung

Die Erfindung betrifft ein System umfassend ein über einen Fußschalter steuerbares medizintechnisches Gerät und einen Fußschalter.

In der Medizintechnik sind Fußschalter zur Steuerung von medizintechnischen Geräten gängig. Durch Fußschalter ist es bspw. möglich, den Aktivierungszustand eines handgeführten Instruments zu steuern, ohne dass dazu die Führungshand selbst oder auch nur ein Finger davon bewegt werden müsste. Das medizinische Personal kann sich so allein auf die Führung des Instruments per Hand konzentrieren und den Aktivierungszustand des Instruments losgelöst davon per Fuß steuern. Der Aktivierungszustand muss dabei nicht auf das Ein- und Ausschalten einer bestimmten Funktion des handgeführten Instruments beschränkt sein, sondern kann auch eine stufenlose Einstellbarkeit umfassen. Beispiele für medizintechnische Geräte mit entsprechender Fußsteuerung sind Zahnarztinstrumente, wie Bohrer, HF-Generatoren für die Hochfrequenz-Chirurgie, Ultraschall-Generator für Ultraschallskalpelle und/oder Lasersysteme.

Fußschalter können mit dem zu steuernden medizintechnischen Gerät per Kabel verbunden sein. Um die Bewegungsfreiheit des medizintechnischen Personals bspw. in einem Operationssaal nicht einzuschränken oder zu behindern, sind aber auch Fußschalter bekannt, die drahtlos Steuerungssignale an das zu steuernde medizintechnische Gerät übertragen. Ein geeigneter Empfänger für die Steuerungssignale muss dann entweder unmittelbar in das medizintechnische Gerät integriert sein, oder es ist ein geeigneter externer Empfänger an dem Signaleingang, an dem bspw. auch ein kabelgebundener Fußschalter angeschlossen werden kann, vorzusehen.

Um einen problemlosen Einsatz eines über einen Fußschalter steuerbares medizintechnisches Gerät sicherzustellen, ist bei einer drahtlosen Übertragung von Steuerungssignalen erforderlich, dass die Signalstärke der am medizintechnischen Gerät empfangenen Steuerungssignal ausreichend hoch ist, dass die Steuerungssignale eindeutig identifiziert und anschließend umgesetzt werden können.

Es hat sich gezeigt, dass bei der Verwendung medizintechnischer Geräte bspw. in Praxen oder Operationssälen diverse Störungsquellen für die drahtlose Übertragung von Steuerungssignalen zwischen einem Fußschalter und dem zugeordneten medizintechnischen Gerät ergeben können, die einen ordnungsgemäßen Betrieb eines medizintechnischen Geräts über den Fußschalter einschränken oder gar unmöglich machen.

Mögliche Störungsquellen sind andere, Funksignale aussendende Geräte, die entweder auf der gleichen Frequenz senden und somit die Steuerungssignale überlagern oder aber zumindest zu einem erhöhten Rauschen führen, von dem sich die Steuerungssignale nicht mehr ausreichend abheben. Auch können mobile Instrumententische, die häufig aus Metall gefertigt sind, oder rollbare technische Geräte die Funkstrecke zwischen Fußschalter und dem zugeordneten medizintechnischen Gerät stören.

Dabei können insbesondere Störungen, die sich während der grundsätzlichen Verwendung des medizintechnischen Geräts, bspw. aufgrund des Verschiebens von Instrumententischen oder dem Einschalten anderer technischer Geräte ergeben, kritisch sein. Auch ist in solchen Situationen - häufig während der Behandlung eines Patienten - eine ausführliche Fehlersuche meist nicht möglich.

Aufgabe der vorliegenden Erfindung ist es ein System umfassend ein über einen Fußschalter steuerbares medizintechnisches Gerät und einen Fußschalter zu schaffen, bei dem die aus dem Stand der Technik bekannten Probleme wenigstens abgemildert sind.

Gelöst wird diese Aufgabe durch ein System gemäß dem Hauptanspruch. Vorteilhafte Weiterbildungen sind Gegenstand der abhängigen Ansprüche.

Demnach betrifft die Erfindung ein System umfassend ein über einen Fußschalter steuerbares medizintechnisches Gerät und einen Fußschalter, wobei der Fußschalter einen Transmitter und das medizintechnische Gerät einen Receiver zur drahtlosen Übermittlung von eindeutig zuordenbaren Signalen zur Steuerung vom Fußschalter zum medizintechnischen Gerät umfasst, wobei der Receiver des medizintechnischen Geräts zur Messung der Signalstärke von Signalen des Fußschalters und das medizintechnische Gerät zur Ausgabe einer Warnung, sofern die gemessene Signalstärke oder das Signal-Rausch-Verhältnis unter einem vorgegebenen Grenzwert liegt, ausgebildet ist.

Die Erfindung hat erkannt, dass durch Überwachung der Signalstärke der vom Fußschalter bzw. dessen Transmitter ausgesendeten Signale am medizintechnisches Gerät zumindest sichergestellt werden kann, dass eine während der Verwendung des medizintechnischen Gerätes plötzlich auftretende Störung der Funkverbindung zwischen Fußschalter und medizintechnischem Gerät unmittelbar als solche erkannt werden kann. Eine entsprechende ausgegebene Warnung ermöglicht außerdem häufig eine unmittelbare Ermittlung der Störungsquelle, wie bspw. das Verschieben eines Instrumententisches oder das Einschalten eines bestimmten Gerätes, auf das dann geeignet reagiert werden kann, bspw. indem der Instrumententisch, das fragliche Gerät oder der Fußschalter verschoben wird, um die Störung soweit zu reduzieren, dass die Signalstärke für einen störungsfreien Betrieb wieder ausreicht.

Auch wenn mithilfe der Erfindung also keine absolut störungsfreie Kommunikation zwischen Fußschalter und medizintechnischem Gerät unabhängig von äußeren Umständen sichergestellt werden kann, so wird bei dem erfindungsgemäßen System doch unmittelbar eine Warnung ausgegeben, sobald eine Störung der fraglichen Kommunikation vorliegt. Das ermöglicht ein unmittelbares Eingreifen, bspw. zur Beseitigung der Störungsquelle, und verhindert so einen für den Benutzer des medizintechnischen Gerätes ansonsten plötzlich auftretenden und nicht unmittelbar erklärlichen Ausfall. Letzteres ist bei medizintechnischen Geräten und deren üblicher Verwendung in Operationssälen oder Behandlungsräumen äußerst unerwünscht und kann sogar erhebliche gesundheitliche Konsequenzen für einen Patienten haben.

Alternativ oder zusätzlich zur Ermittlung der eigentlichen Signalstärke und dem Abgleich mit einem vorgegebenen Grenzwert kann auch das Signal-Rausch-Verhältnis für die Signale vom Fußschalter überwacht werden. Das Signal-Rausch-Verhältnis gibt an, wie deutlich sich das Nutzsignal, hier also das Signal vom Fußschalter, vom Hintergrundrauschen abhebt, was unmittelbar Auswirkung auf die Möglichkeit hat, Informationen sicher aus dem Signal extrahieren zu können. Bei digitalen Signalen besteht häufig ein Zusammenhang zwischen dem Signal-Rausch-Verhältnis und der Fehlerrate.

Es ist bevorzugt, wenn der Transmitter des Fußschalters und der Receiver des medizintechnischen Geräts jeweils Teil eines Transceivers jeweils umfassend einen Transmitter und einen Receiver zur bidirektionalen Kommunikation zwischen Fußschalter und medizintechnischem Gerät sind. Dadurch ist eine bidirektionale Kommunikation zwischen Fußschalter und medizintechnischem Gerät möglich, die bspw. dazu genutzt werden kann, den Fußschalter mit dem medizintechnischen Gerät zu koppeln, also den Fußschalter derart mit dem medizintechnischen Gerät logisch zu verknüpfen, dass ausschließlich dessen Signale ausschließlich von dem betroffenen medizintechnischen Gerät verarbeitet werden.

Sind sowohl beim Fußschalter als auch beim medizintechnischen Gerät Transceiver vorgesehen, ist bevorzugt, wenn der Transceiver des Fußschalters zur Messung der Signalstärke von Signalen des medizintechnischen Geräts und der Fußschalter zur Ausgabe einer Warnung, sofern die gemessene Signalstärke oder das Signal-Rausch-Verhältnis unter einem vorgegebenen Grenzwert liegt, ausgebildet ist. Auf diese Weise kann die Überprüfung der Signalstärke und/oder dem Signal-Rausch-Verhältnis und somit der störungsfreien Kommunikation zwischen Fußschalter und medizintechnischem Gerät redundant erfolgen, womit die Gewissheit unmittelbar auf eine Störung dieser Kommunikation hingewiesen zu werden, gesteigert werden. Auch eine Fehlfunktion eines der beiden Transceiver kann so zumindest als mögliche Störung der Kommunikation erkannt werden. In einem solchen Fall würde nämlich bspw. lediglich eines der beiden Geräte eine Warnung ausgeben.

Es kann vorteilhaft sein, wenn der Transceiver des Fußschalters und/oder des medizintechnischen Geräts dazu ausgebildet ist, auf ein vom Transceiver des medizintechnischen Geräts oder des Fußschalters empfangenes Ping-Signal ein Signal auszusenden, anhand derer der Receiver des medizintechnischen Geräts oder des Fußschalters die Signalstärke ermitteln kann. Bei einem "Ping-Signal" handelt es sich um ein mit dem Zweck ausgesendetes Signal, von dem Kommunikationspartner eine Rückantwort zu erhalten, die in der Regel ohne spezifischen Inhalt ist und lediglich der Messung der erfindungsgemäßen Signalstärke und/oder dem Signal-Rausch-Verhältnis ermöglicht. Selbstverständlich ist es auch möglich, aus der Rückantwort auf ein Ping-Signal weitere Informationen, wie bspw. Signalumlaufzeiten, zu bestimmen.

Alternativ oder zusätzlich ist weiterhin bevorzugt, wenn der Fußschalter und/oder das medizintechnische Gerät dazu ausgebildet sind, unabhängig von einer erforderlichen Steuerung des medizintechnischen Geräts regemäßig Signale auszusenden, anhand derer der Receiver des medizintechnischen Geräts oder des Fußschalters die Signalstärke ermitteln kann.

Mit beiden vorstehend genannten Varianten kann sichergestellt werden, dass Signale zwischen Fußschalter und medizintechnischem Gerät ausgetauscht werden, auch wenn bspw. aufgrund einer Nicht-Betätigung des Fußschalters keine tatsächlichen Steuerungssignale gesendet werden müssen. Bei der beschriebenen Variante mit Ping-Signal kann die Elektronik des einen Gerätes, welches lediglich auf das Ping-Signal reagieren muss, häufig sehr einfach ausgestaltet werden, was sich insbesondere für den Fußschalter anbietet. Das beschriebene regelmäßige Aussenden von Signalen hat sich als besonders robust erwiesen.

Der Fußschalter und/oder das medizintechnische Gerät können zur Ausgabe einer Warnung optische und/oder akustische Warnelemente aufweisen. Dabei ist für den Fußschalter insbesondere ein akustisches Warnelement bevorzugt, während das medizintechnische Gerät vorzugsweise sowohl optische als auch akustische Warnelemente aufweist. Ein optisches Warnelement kann bspw. eine Warnlampe bzw. eine Warn-LED sein; bei dem akustischen Warnelement kann es sich um einen Tongenerator handeln.

Es ist alternativ oder zusätzlich bevorzugt, wenn der Fußschalter und/oder das medizintechnische Gerät einen optischen Indikator zur Anzeige der Signalstärke aufweist. Der optische Indikator kann dabei bspw. als Balkenanzeige, wie sie von Mobiltelefonen bekannt ist, ausgestaltet sein. Ein entsprechender Indikator kann helfen, Probleme in der Kommunikation zwischen Fußschalter und medizintechnischem Gerät aufzuzeigen, bevor es zu einer tatsächlichen Störung kommt, die in einer Warnung resultiert.

Der Fußschalter kann einen Energiespeicher, vorzugsweise einen Akkumulator, für einen vollständig kabellosen Betrieb umfassen.

Vorzugsweise sind der Fußschalter und das medizintechnische Gerät zur Kommunikation gemäß einem Bluetooth-Standard ausgebildet.

Bei dem zu steuernden medizintechnischen Gerät kann es sich bspw. um einen ein HF-Generator, insbesondere für die Endoskopie, ein Ultraschall-Generator und/oder ein Lasersystem handeln.

Die Erfindung wird nun anhand einer vorteilhaften Ausführungsform unter Bezugnahme auf die beigefügte Zeichnung beispielhaft beschrieben. Es zeigt:

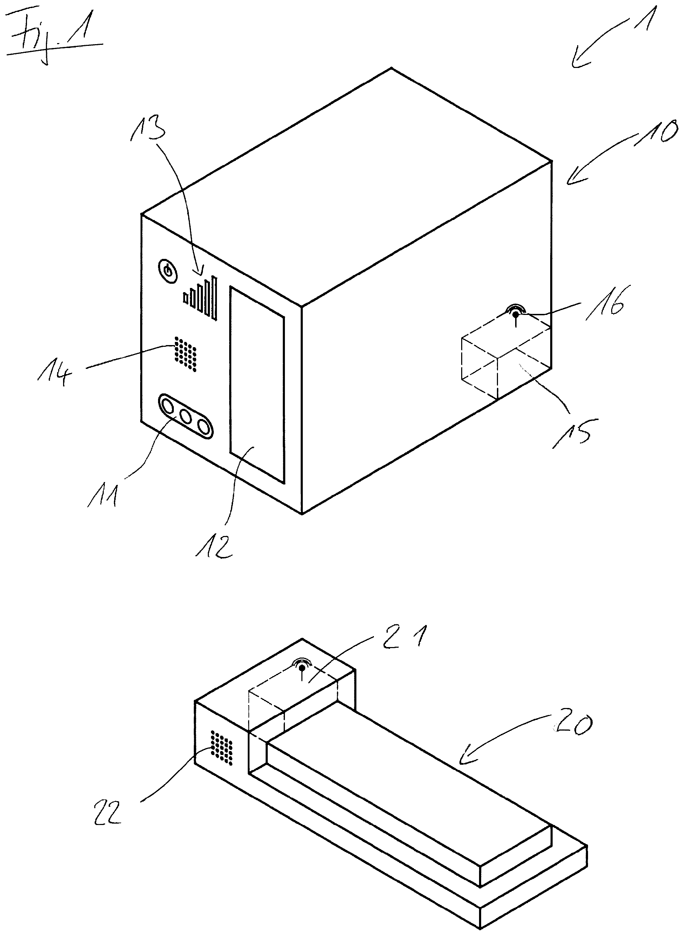

- Figur 1:: ein erstes Ausführungsbeispiel eines erfindungsgemäßen Systems.

In Figur 1 ist ein erstes Ausführungsbeispiel eines erfindungsgemäßen Systems 1 umfassend ein medizintechnisches Gerät 10 und einen Fußschalter 20, die drahtlos miteinander verbunden sind.

Bei dem medizintechnischen Gerät 10 handelt es sich um einen HF-Generator, an dessen Anschlüsse 11 sich ein Elektroskalpell (nicht dargestellt), bspw. auch für die minimalinvasive Chirurgie, anschließen lässt. Die Einstellungen für des HF-Generators lassen sich am berührungsempfindlichen Display 12 ablesen, aber auch einstellen.

Neben dem Display 12 ist noch eine Balkenanzeige 13, sowie ein Tongenerator als akustisches Warnelement 14 vorgesehen. Innenliegend im medizintechnischen Gerät 10 ist ein Transceiver 15 mit ebenfalls innenliegender Antenne 16 vorgesehen. Der Transceiver 15 umfasst einen Transmitter und einen Receiver und ist zum Senden und Empfangen von Signalen gemäß einem Bluetooth-Standard ausgebildet.

Der Fußschalter 20 ist batteriebetrieben und weist ebenfalls einen Transceiver 21 auf, der zum Senden und Empfangen von Signalen gemäß demselben Bluetooth-Standard wie der Transceiver 15 des medizintechnischen Gerätes 10 ausgebildet ist. Außerdem weist der Fußschalter 20 noch einen Tongenerator als akustisches Warnelement 22 auf.

Sowohl der Transceiver 15 des medizintechnischen Gerätes 10 als auch der Transceiver 21 des Fußschalters 20 sind dazu ausgebildet miteinander zu kommunizieren. Insbesondere werden Signale über die Betätigungen des Fußschalters 20 zwischen diesen beiden Transceiver 15, 21 übertragen, sodass das medizintechnische Gerät 10 entsprechend der Stellung des Fußschalters 20 gesteuert wird.

Beide Transceiver 15, 21 sind dabei weiterhin dazu ausgebildet, die Signalstärke sowie das Signal-Rausch-Verhältnis der zwischen ihnen ausgetauschten Signale zu ermitteln. Die von dem Transceiver 15 des medizintechnischen Gerätes ermittelte Signalstärke wird unmittelbar über die Balkenanzeige 13 zur Anzeige gebracht.

Darüber hinaus vergleichen sowohl der Transceiver 15 des medizintechnischen Gerätes 10 als auch der Transceiver 21 des Fußschalters 20 die ermittelte Signalstärke und das ermittelte Signal-Rausch-Verhältnis mit jeweils vorgegebenen Grenzwerten. Wird ein Grenzwert unterschritten, bedeutet dies eine abgebrochene oder schlechte Verbindung zwischen den beiden Transceivern 15, 21, woraufhin eine akustische Warnung über den jeweiligen Tongenerator 14, 22 ausgegeben wird. Zusätzlich kann die Balkenanzeige 13 des medizintechnischen Gerätes 10 in einer anderen Farbe als gewöhnlich erleuchtet werden und/oder blinken.

Um sicherzustellen, dass der Transceiver 21 des Fußschalters 20 die fragliche Ermittlung und Überprüfung der Signalstärke bzw. des Signal-Rausch-Verhältnisses regelmäßig durchführen kann, sendet der Transceivers 15 des medizintechnischen Gerätes 10 in regelmäßigen Abständen Signale an den Fußschalter 20, die von dessen Transceiver 21 empfangen und entsprechend verarbeitet werden.

Bei diesen vom Transceiver 15 des medizintechnischen Gerätes 10 regelmäßig ausgesendeten Signalen handelt es sich um Ping-Signale, die neben der Überprüfung der Signalstärke bzw. des Signal-Rausch-Verhältnisses durch den Fußschalter 20 gleichzeitig eine Aufforderung an dessen Transceiver 21 darstellen, ebenfalls ein Signal auszusenden, unabhängig davon, ob eine Betätigung des Fußschalters, welche alternativ ein Signal auslösen würde, vorliegt oder nicht. Auf diese Weise kann auch der Transceiver 15 des medizintechnischen Gerätes 10 regelmäßig die Signalstärke und das Signal-Rausch-Verhältnis überprüfen, und - wie beschrieben - eine Warnung ausgeben, sobald einer der beiden Werte unter einen jeweils vorgegebenen Grenzwert absinkt.

Bei den vorstehend erwähnten Ping-Signalen kann es sich insbesondere um in dem verwendeten Bluetooth-Standard vorgesehene Ping-Signale handeln.

## Patentansprüche

1. System (1) umfassend ein über einen Fußschalter (20) steuerbares medizintechnisches Gerät (10) und einen Fußschalter (20), wobei der Fußschalter (20) einen Transmitter und das medizintechnische Gerät (10) einen Receiver zur drahtlosen Übermittlung von eindeutig zuordenbaren Signalen zur Steuerung vom Fußschalter (20) zum medizintechnischen Gerät (10) umfasst,
**dadurch gekennzeichnet, dass**
der Receiver des medizintechnischen Geräts (10) zur Messung der Signalstärke von Signalen des Fußschalters (20) und das medizintechnische Gerät (10) zur Ausgabe einer Warnung, sofern die gemessene Signalstärke oder das Signal-Rausch-Verhältnis unter einem vorgegebenen Grenzwert liegt, ausgebildet ist.

2. System nach Anspruch 1,
**dadurch gekennzeichnet, dass**
der Transmitter des Fußschalters (20) und der Receiver des medizintechnischen Geräts (10) jeweils Teil eines Transceivers (15, 21) jeweils umfassend einen Transmitter und einen Receiver zur bidirektionalen Kommunikation zwischen Fußschalter (20) und medizintechnischem Gerät (10) sind.

3. System nach Anspruch 2,
**dadurch gekennzeichnet, dass**
der Transceiver (21) des Fußschalters (20) zur Messung der Signalstärke von Signalen des medizintechnischen Geräts (10) und der Fußschalter (20) zur Ausgabe einer Warnung, sofern die gemessene Signalstärke oder das Signal-Rausch-Verhältnis unter einem vorgegebenen Grenzwert liegt, ausgebildet ist.

4. System nach Anspruch 2 oder 3,
**dadurch gekennzeichnet, dass**
der Transceiver (21, 15) des Fußschalters (20) und/oder des medizintechnischen Geräts (10) dazu ausgebildet ist, auf ein vom Transceiver (15, 21) des medizintechnischen Geräts (10) oder des Fußschalters (20) empfangenes Ping-Signal ein Signal auszusenden, anhand derer der Transceiver (15, 21) des medizintechnischen Geräts (10) oder des Fußschalters (20) die Signalstärke ermitteln kann.

5. System nach einem der vorhergehenden Ansprüche,
**dadurch gekennzeichnet, dass**
der Fußschalter (20) und/oder das medizintechnische Gerät (10) dazu ausgebildet sind, unabhängig von einer erforderlichen Steuerung des medizintechnischen Geräts (10) regemäßig Signale auszusenden, anhand derer der Receiver des medizintechnischen Geräts (10) oder des Fußschalters (20) die Signalstärke und/oder das Signal-Rausch-Verhältnis ermitteln kann.

6. System nach einem der vorhergehenden Ansprüche,
**dadurch gekennzeichnet, dass**
der Fußschalter (20) und/oder das medizintechnische Gerät (10) zur Ausgabe einer Warnung optische und/oder akustische Warnelemente (14, 22) aufweist.

7. System nach einem der vorhergehenden Ansprüche,
**dadurch gekennzeichnet, dass**
der Fußschalter (20) und/oder das medizintechnische Gerät (10) einen optischen Indikator (13) zur Anzeige der Signalstärke aufweist.

8. System nach einem der vorhergehenden Ansprüche,
**dadurch gekennzeichnet, dass**
der Fußschalter (20) einen Energiespeicher, vorzugsweise einen Akkumulator, zum kabellosen Betrieb umfasst.

9. System nach einem der vorhergehenden Ansprüche,
**dadurch gekennzeichnet, dass**
der Fußschalter (20) und das medizintechnische Gerät (10) zur Kommunikation gemäß einem Bluetooth-Standard ausgebildet sind.

10. System nach einem der vorhergehenden Ansprüche,
**dadurch gekennzeichnet, dass**
das zu steuernde medizintechnische Gerät (10) ein HF-Generator, insbesondere für die Endoskopie, ein Ultraschall-Generator und/oder ein Lasersystem ist.
